# EUROPEAN PATENT APPLICATION

(11) **EP 4 497 388 A1**
(43) Date of publication of application: **29.01.2025**
(21) Application number: 23215901.2
(22) Date of filing: 12.12.2023
(51) Int. Cl.: A61B 6/00

(54) **DETERMINING A SCATTER CORRECTION**

(71) Applicant: Siemens Healthineers AG, 91301 Forchheim (DE)
(72) Inventor: Baer-Beck, Matthias, 91080 Spardorf (DE); Bernhardt, Philipp, 91301 Forchheim (DE); Hofmann, Christian, 91058 Erlangen (DE); Obler, Richard, 91052 Erlangen (DE)
(74) Representative: Siemens Healthineers Patent Attorneys

(57) **Abstract**

A method for determining a scatter correction for an x-ray radiation signal recorded by an x-ray detector (2) of an imaging modality based on x-ray radiation,
the method comprising the following method steps:
(a) receiving inside radiation data and outside scattered radiation data, wherein the inside radiation data is data obtained from a directly illuminated partial area (21) of the x-ray detector (2) with a primary x-ray beam (11) directed at an object within an examination area (3), wherein the outside scattered radiation data is data obtained by detecting scattered radiation (12) outside of the directly illuminated partial area (21);
(b) based on the outside scattered radiation data, determining a scatter correction, in particular by estimating a part of the inside radiation data that is due to the scattered radiation (12).

## Description

The invention relates to a method for determining a scatter correction for an x-ray radiation signal recorded by an x-ray detector of an imaging modality based on x-ray radiation, as well as a corresponding computer program, image processing device, imaging modality and use of imaging data.

Independent of the grammatical term usage, individuals with male, female or other gender identities are included within the term.

In x-ray-based imaging, in particular medical imaging, scatter radiation can be an importation source of image degradation. Hence, when using imaging modalities, such as via computed tomography imaging and/or fluoroscopy correcting scatter radiation can play a crucial role for image processing and/or workflow optimization.

In fluoroscopy, an x-ray source and a detector are used, with the object being in between the x-ray source and the detector. Due to partial absorption of x-ray radiation in the object that is typically different for different materials, the directly transmitted x-ray radiation (herein also denoted as primary x-ray beam) can be representative of the internal structure of the object. However, due to scattering of the radiation, also scattered radiation will hit the detector, therefore degrading the image quality. Typically, a collimator is used to only illuminate a region of interest in an object, such as part of a patient. In many cases, due to the collimator, only a partial area of the detector is illuminated. The collimator may reduce the radiation exposure of the object and may also reduce the amount of scattered radiation. Nevertheless, some scattered radiation, in particularly scattered within the object, will normally still hit the partial area of the detector and therefore negatively affect the image quality.

In computed tomography (CT) imaging, a primary x-ray beam, generated by an x-ray source, is directed at an object in an examination area and detected by an x-ray detector at the opposite side of the examination area. Depending on the properties of the object, parts of the primary x-ray beam are absorbed leading to an object-dependent attenuation. Based on the attenuation of the primary x-ray beam, structures of the object can be determined. By varying the projection angle of the primary x-ray beam towards the object, a three-dimensional image of the object may be generated. However, part of the x-ray radiation is scattered which can lead to a detection of the scattered radiation by the x-ray detector. Scattered radiation that is detected by the CT detector can be a cause of image artifacts and, thus, reduced image quality.

One measure to address the problem of scattered radiation in CT imaging may be an anti-scatter grid. Anti-scatter grids are typically based on the assumption that the incidence angle of the primary x-ray beam onto the detector is constant for each projection angle. While anti-scatter grids may be a reasonable measure for typical third-generation CT systems with a rotating gantry, they are typically not useful for a CT scanner geometry with distributed, in particular static non-rotating, x-ray sources. With distributed x-ray sources, the source to detector alignment and therefore the incidence angle of the x-rays within the primary x-ray beam is different for each source of the distributed sources array. The assumption that the incidence angle of the primary x-ray beam onto the detector is constant for each projection angle does thus not work any longer, usually making corresponding anti-scatter grids unfeasible. Accordingly, scattered radiation can in particular be a problem for scanner geometries with distributed x-ray sources.

In order to address the problem of scattered radiation in CT, it has been proposed to provide a scatter estimation and correction by simulating the physical process of x-ray interaction, e.g. by Monte Carlo simulations, and/or to use scatter kernels to estimate the scattered radiation directly for the primary x-ray beam. Corresponding correction and estimation methods are, for example, described by Rührnschopf, E. P., & Klingenbeck, K. in "A general framework and review of scatter correction methods in x-ray cone-beam computerized tomography" (2011), Part 1: scatter compensation approaches. Medical physics, 38(7), 4296-4311; and Part 2: scatter estimation approaches. Medical physics, 38(9), 5186-5199. Although direct Monte Carlo-based methods have the potential to be quite accurate, these methods have the disadvantage to be computational complex and time consuming. This typically prevents their direct usage in clinical CT image reconstruction.

It is therefore an object of the invention to address at least some of the above-mentioned problems, in particular to provide a way to estimate and/or correct the influence of scattered radiation on image data from x-ray-based imaging.

This object is met or exceeded by a method according to claim 1, a computer program according to claim 12, an image processing device according to claim 13, a x-ray-based imaging modality according to claim 14, and a use according to claim 15. Further advantages and features result from the dependent claims, the description and the attached figures.

According to a first aspect of the invention, a method for determining a scatter correction for an x-ray radiation signal recorded by an x-ray detector of an imaging modality based on x-ray radiation is provided. The method comprises the following method steps:
(a) receiving inside radiation data and outside scattered radiation data, wherein the inside radiation data is data obtained from a directly illuminated partial area of the x-ray detector with a primary x-ray beam directed at an object within the examination area, wherein the outside scattered radiation data is data obtained by detecting scattered radiation outside of the directly illuminated partial area;
(b) based on the outside scattered radiation data, determining the scatter correction, in particular by estimating a part of the inside radiation data that is due to the scattered radiation.

According to an embodiment, the method further comprises as part of receiving the inside radiation data and the outside scattered radiation data:
- directly illuminating the partial area of the x-ray detector with the primary x-ray beam directed at the object within the examination area, and detecting x-ray radiation within the partial area as inside radiation data;
- detecting scattered radiation with a part of the detector outside of the directly illuminated partial area, in particular with at least some of the detector elements of the detector outside of the directly illuminated partial area, as outside scattered radiation data.

In the context of this invention, the term "scatter correction" is to be interpreted broadly. It may generally refer to any kind of information or action that may be helpful or feasible to account for scattered radiation in detected image data. For example, the scatter correction may comprise information about the part of recorded radiation data that is due to scattered radiation. For example, the scatter correction may comprise a relative amount, e.g. a percentage, of the total detected radiation data, or an absolute amount of detected radiation, e.g. determined for a particular measurement. The scatter correction may comprise an action, such as subtracting an intensity profile corresponding to scattered radiation from the inside radiation data.

The examination area may generally be designed such that the object can be placed in the examination area. The object may be part of a larger object or of a subject. A subject may, for example, be a human being, such as a patient, or an animal. For example, the object may be an anatomical part, e.g. a limb or an organ, of the subject. The object may be any other kind of object, e.g. luggage. For example, a table, in particular a patient table, may be provided that is at least partially located or locatable inside the examination area. The table may be suitable to place the object and/or the subject on the table. The table may be movable, e.g. to move the subject or the object that is part of the subject into the examination area.

The x-ray detector may comprise detector elements. Detector elements may also be denoted as detector pixels or be representative of detector pixels. The detector resolution and/or the detector grid size may be determined (partially) by the detector elements and their respective distance to each other.

The imaging modality comprises at least one x-ray source. Hence an imaging modality based on x-ray radiation is an imaging modality that uses x-rays to generate images. Examples for such imaging modalities may be computed tomography systems, fluoroscopes, and projectional radiographs. The at least one x-ray source, in particular the x-ray sources, may be configured to emit the primary x-ray beam. An x-ray source may also be denoted as x-ray generator. The at least one x-ray source is preferably directed at the examination area and the x-ray detector, at least part of which x-ray detector is beyond the examination area. For example, the x-ray detector may be based on an x-ray tube as known from the state of the art.

The partial area comprises a part of the detecting area of the x-ray detector. Hence some part of the x-ray detector is not directly illuminated by the primary x-ray beam. "Directly illuminating" may be understood such that x-rays that illuminate directly are directly transmitted through the object or past the object (e.g., depending on the object size and shape) to the detector. On the other hand, scattered radiation is not part of the primary x-ray beam that directly illuminates the partial area. However, typically, some scattered radiation reaches the directly illuminated partial area as well. Hence, typically, the detected signal of the part of the detecting area of the detector is based on radiation from the primary x-ray beam and on scattered radiation. The primary x-ray beam may, for example, be an x-ray cone beam.

In the context of this disclosure, the x-ray radiation data that is recorded by the detector within the partial area is denoted as inside radiation data, i.e. radiation data that is detected inside the partial area. The scattered radiation that is detected by the detector within the partial area may be denoted as inside scattered radiation data. Generally, in the context of this application, "x-ray radiation" may also be denoted as simply "radiation."

Radiation may be scattered from within the object or, in some cases, from other scatter sources. The outside scattered radiation data may be based on the measurement of all the detector area outside the partial area. Alternatively, the outside scattered radiation data may be based on only a part of the detector area outside the partial area. For example, the outside scattered radiation data may be based on only this part of the detector area outside the partial area that is closest to the partial area. Since the detector area outside the partial area is not directly illuminated by the primary x-ray beam, this outside detector area can only be reached by scattered radiation. Accordingly, it may be assumed that any radiation data reaching this outside detector area is essentially, in particular exclusively, scattered radiation. In the context of this disclosure, the x-ray radiation detected outside the partial area is denoted as "outside scattered radiation data." Preferably the inside radiation data and the outside scattered radiation data is detected essentially simultaneously and/or during the same measurement. It may generally also be feasible to acquire these data separately, e.g. in consecutive measurements. However, it is typically more advantageous to acquire the data at the same time to save time and reduce the applied x-ray dose.

The scatter correction is determined based on the outside scattered radiation data. Hence, the measured scatter intensity, measured outside the directly illuminated partial area, may be used to estimate the fraction of scattered radiation that reaches the detector also in the region of the primary x-ray beam. Typically, in the state of the art, only the region of the primary x-ray beam is used for the medical image and/or for image reconstruction. If the corresponding data is not corrected for scattered radiation the scattered radiation may produce strong artifacts in images which can significantly decrease the achievable image quality. Within this invention it is taken advantage of the fact that the scattered radiation can reach both the detector within the partial area and outside the partial area. It has been recognized that, typically, there is a correlation between the scattered radiation inside the directly illuminated partial area and outside the partial area. In other words, the scattered radiation outside the partial area is typically characteristic for the scattered radiation inside the directly illuminated partial area at least in some way. Due to this, the measured scatter intensities outside the partial area can be assumed to contain the signature of the scatter intensities inside the partial area, in particular also within the object shadow. Hence, the scatter intensity measured in the regions outside the primary x-ray beam can be used to estimate the scatter intensities even inside the partial area. Advantageously, radiation data measured by the detector area which is located outside the primary x-ray beam may thus be used to estimate the fraction of scattered radiation detected by the detector area that lies inside the partial area illuminated by the primary x-ray beam, and which is used for image reconstruction. Advantageously, particularly long computation times for estimating the scatter correction may be avoided that might occur, if the scatter correction is entirely based only on simulation and/or theoretical calculation. On the other hand, the measurement workflow and process does not have to be impaired, e.g. by providing additional sensors for determining the scattered radiation or by performing additional measurements cycles, since the required data can be detected simultaneously (i.e., by the detector area outside the partial area) with the actual data of the primary x-ray beam. The scatter correction may in particular be determined by estimating the part of the inside radiation data that is due to scattered radiation. Estimating the part of the inside radiation data that is due to scattered radiation may comprise estimating an amount and/or a distribution of the inside radiation data that is due to scattered radiation.

According to an embodiment, the imaging modality is a computed tomography (CT) system, in particular a static computed tomography system, wherein the x-ray detector comprises an array of detector elements arranged at least partially around an examination area, wherein the partial area comprises a subset of the detector elements, wherein the outside scattered radiation data is detected with at least some of the detector elements outside of the directly illuminated partial area. The examination area of the computed tomography system may generally be designed such that the object can be placed in the examination area. The computed tomography system may comprise a gantry, the gantry comprising the at least one x-ray source and the detector elements of the x-ray detector. The examination area may in particular be located at least partially within the gantry. In the context of this invention, a static computed tomography system is in particular a computed tomography system having x-ray sources and detector elements that are non-rotating during a computed tomography scan. The gantry may be configured such that it does not rotate around the examination area during a computed tomography measurement. Accordingly, the x-ray detector may be a static x-ray detector. Advantageously, rotational forces may be avoided with a static computed tomography system. The detector elements of the x-ray detector are arranged at least partially around the examination area. The detector elements being arranged partially around the examination area may be understood such, that the detector elements are only distributed at parts of the circumference, e.g. a ring-shaped circumference, of the examination area and/or that there is a gap in the circumference of the examination area, the gap having no or fewer detector elements than other parts of the circumference. For example, the detector elements may be arranged according to an arc-shape and/or according to a partial ring. The detector elements may be also distributed over a circular or partially circular arrangement around the examination area. Preferably, an x-ray detector is used that has an extension of the arrangement of detector elements that is wider, in particular at least 5%, preferably at least 10%, wider, than the directly illuminated partial area of the detector. In particular, the detector elements may be distributed over an arrangement with an angle that is greater than the angle of the primary x-ray beam's cone-shaped form. Preferably, the detector elements may be distributed over 360°. In other words, the detector elements may be arranged completely around the examination area.

In the context of this invention, the direction perpendicular to the arrangement of the detector elements of the CT system may be referred to as axial direction. In particular, the axial direction may be perpendicular to an area spanned by the arrangement of the detector elements. The axial direction may be the axial direction of a (partial) ring-shaped arrangement of the detector elements. The axial direction may correspond to a z-direction in cartesian coordinates. A distribution in the axial direction may be comprised even if a general shape, such as a ring-shaped or annular arrangement of detector elements is referred to. A direction that is perpendicular to the axial direction may be referred to as radial direction. In particular, the radial direction may be direction from the centre of the examination area towards any one of the detector elements. The radial direction may correspond to an x,y-direction. Furthermore, a circumferential direction may be defined. The circumferential direction is a direction following the arrangement of the detector elements around the examination area. In particular, for a ring-shaped detector, the circumferential direction may follow the annular form of the ring.

The arrangement of detector elements of the computed tomography system may comprise rows and/or columns of detector elements. Rows of detector elements may be detector elements that are arranged one after the other in the circumferential direction. Rows of detector elements may be detector elements that are arranged one after the other in the axial direction. Hence, the distribution of the detector elements may extend in the axial direction.

The computed tomography system may comprise at least one x-ray source, preferably multiple x-ray sources. The at least one x-ray source, in particular the x-ray sources, may be configured to emit the primary x-ray beam. For example, distributed x-ray sources may be used, wherein multiple x-ray sources are distributed over a circular or partially circular arrangement around the examination area. The x-ray sources of the computed tomography system may be distributed around the examination area. For example, the x-ray sources may be distributed over 360°. The x-ray sources may be part of an x-ray source array. The x-ray source array may be ring-shaped. The x-ray source array may be a static x-ray source array, in particular corresponding to the static computed tomography system. Hence, the x-ray sources may be stationary during operation. In other words, in case of a static computed tomography system, the x-ray sources are in particular not rotated around the examination area during a scan. Preferably, both, the x-ray sources of the computed tomography system and the detector elements may be distributed around the examination area. The x-ray sources may be oriented towards the detector elements, in particular towards the detector elements on the opposite side of the examination area. In particular the x-ray sources, the examination area, and the x-ray detector may be arranged such that the primary x-ray beam can reach the detector elements of the x-ray detector in a straight line through the examination area. A rotational scan of the examination area may be achieved by selectively activating x-ray sources. For example, the x-ray sources may be selectively activated by electronic switching.

The partial area comprises a subset of the detector elements. Hence some of the detector elements are not directly illuminated by the primary x-ray beam. "Directly illuminating" may be understood such that x-rays that illuminate directly are directly transmitted through the object or past the object (e.g., depending on the object size and shape) to the detector elements. On the other hand, scattered radiation is not part of the primary x-ray beam that directly illuminates the partial area. However, typically, some scattered radiation reaches the directly illuminated partial area as well. Hence, typically, the detected signal of the subset of detector elements comprises radiation from the primary x-ray beam and scattered radiation. The primary x-ray beam may in particular be an x-ray cone beam.

According to an embodiment, the partial area of the x-ray detector is a coherent arc-shaped section of the arrangement of detector elements, in particular corresponding to a cone angle of a cone-shaped primary x-ray beam. Hence, the angle of the cone may define the partial area of the x-ray detector that is directly illuminated. The outside scattered radiation data may be based on the measurement of all the detector elements outside the partial area. Alternatively, the outside scattered radiation data may be based on only a part of the detector elements outside the partial area. For example, the outside scattered radiation data may be based on only these detector elements outside the partial area that are closest to the partial area.

The proposed scatter correction is particularly advantageous for static computed tomography systems and/or for computed tomography systems with distributed x-ray sources, since no anti-scatter grids can be installed as they are typically used in current state of the are 3rd generation clinical computed tomography scanners with rotating gantry. Furthermore, having an x-ray detector with a wide arc range of detector elements can also be particularly beneficial for both, this method as well as for static computed tomography systems in general.

According to an embodiment, the detector elements of the CT system are arranged around the examination area in a ring-shaped manner, in particular forming a 360° detector. A 360° detector is in particular an x-ray detector that has detector elements arranged all around the examination area. Advantageously, a 360° detector can be advantageous in that more scattered radiation, i.e. from a larger total detector area, can be used to determine the scattered radiation within the partial area. Having more scatter information may allow to determine the scatter correction more accurately. In particular, having a 360° detector can provide information about the object's shape and the scatter properties of the object. The scatter properties may comprise information about the internal structure and materials within the object that affect the scattering of radiation.

According to an embodiment, the imaging modality is a fluoroscope comprising a collimator, wherein the collimation via the collimator defines the partial area on the x-ray detector. The fluoroscope may be or may comprise a c-arm, in particular mobile c-arm. The fluoroscope may comprise an x-ray source and an x-ray detector. The x-ray source and the x-ray detector may be on opposite ends of the c-arm. The collimator may be located between the x-ray source and the x-ray detector, in particular in a straight line between the x-ray source and the x-ray detector. The direct line between the x-ray source and the x-ray detector may be defined to be a longitudinal direction in the context of this invention. The collimator may be located in front of the x-ray source. In particular, the collimator may be located between the x-ray source and the examination area. The collimator may be configured such that a collimation window is adaptable via the collimator. Optionally, for this method, the collimator may be set such that the partial area on the detector that is directly illuminated by the x-rays is smaller than the remaining area of the detector that is not directly illuminated by the x-rays. The amount of collimation may depend on the zoom format that is used for a particular fluoroscopy examination. The collimation may be set automatically or manually. While the collimation may prevent the x-rays from directly hitting the detector outside of the partial area, i.e. outside of the collimation window, scattered radiation, in particular radiation scattered from the examined object (such as part of a patient), may still reach the area outside the partial area. Advantageously, by using the information form outside the partial area, an adequate estimation of the scattered radiation within the partial area may be achieved. This may lead to an improvement of image processing and/or workflow optimization.

According to an embodiment, estimating the part of the inside radiation data that is due to scattered radiation comprises applying a calculation model and/or an algorithm that derives the scattered radiation within the partial area from the outside scattered radiation data. Advantageously, the calculation model or algorithm may be relatively simple, i.e. requiring relatively little computational time, since due to the provided outside scattered radiation data, it is not necessary to simulate the scattering completely from scratch, but the outside scattered radiation data can be used as basis.

According to an embodiment, scattered radiation within the partial area is determined by interpolating from the outside scattered radiation data. For example, a linear interpolation from at least some of the outside scattered radiation data may be applied. The interpolation, in particular linear interpolation, may be based on the detector part, e.g. detector elements, outside the partial area that is closest to the partial area. In other words, the interpolation may be performed from the measured scatter intensities of the detector part that lies just outside the illuminated primary x-ray beam. Advantageously, applying an interpolation may provide a relatively simply way of using the outside scattered radiation data for the determination of the scatter correction.

According to an embodiment, scattered radiation within the partial area is determined by inpainting based on the outside scattered radiation data. Inpainting is a method that is in particular known from image restoration, e.g. of historical images. Inpainting generally describes a process of filling missing or faulty parts of an image to achieve a complete image. In the context of this invention, the scattered radiation across both the outside scattered radiation data and the inside scattered radiation data may be treated as the whole image and the inside scattered radiation data may be the data that is to be completed. The inside scattered radiation data may be treated as a blank, i.e. completely missing data. Alternatively, the inside scattered radiation data may be treated as faulty, such as comprising the inside scattered radiation data and additionally comprising the data caused by the direct illumination with the primary x-ray beam. Hence, via the inpainting, the inside scattered radiation data may be estimated in order to determine the scatter correction. For example, structural inpainting may be applied. Additionally or alternatively, texture inpainting may be applied. The inpainting may be based on a machine learning algorithm. For example, the inpainting may be based on optical flow and/or optical diffusion.

According to an embodiment, a structure of scattered radiation outside the partial area is determined from the outside scattered radiation data, and the structure of scattered radiation is interpolated and/or inpainted into the partial area in order to estimate the part of the inside radiation data that is due to scattered radiation. For example, the signal strength from right outside the border of the partial area and/or further signal strengths may be determined. Optionally, the structure of scattered radiation outside the partial area may be or may be represented by at least one scatter line profile or a two-dimensional scatter distribution. The two-dimensional scatter distribution may correspond to the two-dimensional area of the x-ray detector outside the partial area. For example, the structure may comprise at least one pattern and/or at least one trend. For example, a trend may be used by progressively transferring information inwards into the partial area. For example, a structural inpainting may be applied based on the at least one trend. For example, the pattern may be a periodic pattern or a pattern having periodic aspects. Periodic aspects may, for example, be some parts of the pattern reoccurring regularly. For example, frequency and/or spatial domain information from the outside scattered radiation data may be combined to fill in information for the inside radiation data. For example, a textural inpainting may be applied based on the periodic pattern. Advantageously, this embodiment uses the realisation, that the scatter intensities from outside the partial area may typically comprises a signature that may be used to derive the scatter intensities inside the partial area. For example, information from detector elements of a detector, the detector spanning at least 270°, preferably a 360° detector, outside the partial area may be used to perform the inpainting. Hence, advantageously, a wide area of the detector, in particular of the detector elements, providing information of a scatter profile over a correspondingly large area, may be used as basis of the scatter correction. A scatter line profile may be a line plot of the detector signal outside the partial area. Advantageously, the scatter line profile may be an easy to apply way to extract scatter information, in particular to extract information about a trend and/or strength of the scatter signal. The at least one scatter line profile may follow a straight line from a point outside of the partial area to the border of the partial area. The scatter line profile may be oriented perpendicular to a side of the partial area, in particular to a side (in particular border) of the partial area the scatter line profile ends at. Preferably, at least two scatter line profiles, in particular on opposing sides of the partial area, may be used for interpolation. The two scatter line profiles on opposing sides may be oriented parallel with respect to each other. The at least one scatter line profile may be averaged over scatter data in a direction perpendicular to the direction of the line profile.

According to an embodiment, the signal is interpolated from at least four different sides of the partial area. This embodiment may in particular be advantageous for fluoroscopy. In many cases, a collimation in fluoroscopy is determining a partial area that is limited in all directions perpendicular to the longitudinal direction. Accordingly, there may be scatter information all around the partial area. Advantageously, interpolating from four different sides may thus allow to take into account more information (e.g. as opposed to interpolating only from two sides). By approximating from multiple sides, the degree of freedom of the estimation may be reduced, being limited by actual measured conditions. Hence, the reliability of the scatter estimation may be greater. For example, the signal may be interpolated from two pairs of opposite sides. The signal may be interpolated from scatter line profiles from four different sides. Two of the scatter line profiles may be parallel to each other and perpendicular to the other two scatter line profiles. The parallel scatter line profiles may be on opposite sides of the partial area, respectively. Advantageously, scatter information that comprises two-dimensional information (i.e. not only information in one direction but in two directions) may thus be taken into account.

According to an embodiment, at least one pair of signal line plots is interpolated from scatter line plots outside the partial area into the partial area under the condition that the interpolation is continuous and, preferably, differentiable. Preferably, multiple scatter line plots, more preferably at least four scatter line plots, are interpolated under the condition that the interpolation is continuous and, preferably, differentiable. Making the interpolation continuous can be understood such that it is assumed that the scattering at the border of the partial area does not suddenly increase or decrease significantly but that there is a continuous progression of scatter signal. For example, in fluoroscopy, the signal within the collimated partial area tends to be significantly higher than just outside the partial area due to the actual measurement signal of the primary x-ray beam. Hence, for example in a simple approximation, it may be assumed that the strength of the scatter signal just within the partial area continuous at the same strength as the scatter signal just outside the partial area. The interpolation being differentiable may be understood such that the first derivative of the course of the signal, e.g. of a line profile, is continuous. Since, due to a partially random or chaotic nature of the scatter signals the scatter signals tend to be blurrier when compared to the actual measurement signal in the partial area. The blurriness may correspond to low spatial frequencies. Accordingly, assuming a differentiable course of the scatter profile even within the partial area has been found to be a good approximation.

Determining the scatter correction may be further based on at least one additional source of information. For example, the at least one additional source of information may comprise patient data and/or current or previous inside radiation data.

According to an embodiment, determining the scatter correction is further based on previously measured and/or simulated scatter data from within and outside the partial area. Hence, in addition to base the scatter correction on the inside radiation data the previously measured and/or simulated scatter data may be used. The previously measured and/or simulated scatter data may be used to infer how the inside scattered radiation data must likely look for a given outside scattered radiation data. Advantageously, thus, the previously gained information may be used to determine the scatter correction for the present measurement. Additionally or alternatively, determining the scatter correction may be based on the inside radiation data, in particular a distribution and/or signal strength of the inside radiation data. For example, it may be assumed that the scatter intensity inside the partial area is connected, in particular correlated, to the total signal intensity of the inside radiation data. For example, for estimating the inside scattered radiation data, the inside radiation data may be adapted based on the outside scattered radiation data. For example, the signal strength of the inside radiation data may be adapted such that the radiation data is continuous with respect to the outside scattered radiation data. In particular, the signal strength of the inside radiation data may be lowered to match the signal strength of the outside scattered radiation at the border of the partial area. The inside radiation data may be blurred, e.g. based on the blurriness of the outside scattered radiation data and/or based on a set blurring modifier. Blurring may, for example, be applied via a gaussian blurring kernel. The general course of the inside scattered radiation data may be adapted to correspond to the general course of the inside radiation data. For example, it may be assumed that a trend of signal strength of the inside radiation data increasing or decreasing within the partial area corresponds to a trend of the inside scattered radiation data. This may be based on the assumption that a strong overall signal may typically come together with a relatively strong scattering at this position. For example, the inside radiation data may be blurred and adapted to be continuous (and preferably also differentiable) with respect to the outside scattered radiation data. Additionally or alternatively, determining the scatter correction may be based on an geometric measure, in particular thickness, of the object. The geometric measure, in particular thickness, may be estimated. The thickness of the object may be an input parameter. For example, the thickness of the object may be input by a user and/or retrieved from a database. The thickness of the object may, for example be the thickness of a limb or of a torso of a patient. Optionally, the thickness of the object may be derived from the measurement data, e.g. from the inside radiation data and/or from the outside scattered radiation data. For example, by correlating the applied x-ray dose with the received x-ray signal, the thickness of the object may be estimated, in particular based on the assumption that a greater thickness of the object corresponds to a lower received x-ray signal.

According to an embodiment, the scatter correction is determined by comparing the outside scattered radiation data to a database of stored examples of outside scattered radiation data, the stored examples being associated with inside scattered radiation data, respectively, and the scatter correction is determined based on the inside scattered radiation data. For example, the database may comprise a library of reference data, the reference data comprising pairs of outside scattered radiation data and inside scattered radiation data. For example, the library may comprise previously measured and/or simulated data. For example, the data of the library may be measured on test objects and/or phantoms. This embodiment may correspond to a dictionary look-up, wherein the dictionary comprises the library of reference data. For example, the outside scattered radiation data may be compared to the stored examples by looking for the best match between the outside scattered radiation data and the stored examples of outside scattered radiation data. The inside scattered radiation data of the reference data may be used as subtrahend to be subtracted from the inside radiation data. Subtracting the inside scattered radiation data of the reference data from the inside radiation data may then yield scatter corrected data, i.e. scatter corrected inside radiation data. The scatter corrected data may in particular correspond only (the term "only" is to be understood in terms of the accuracy of the applied method) to the x-ray radiation of the primary x-ray beam.

According to an embodiment, comparing the outside scattered radiation data to a database of stored examples of outside scattered radiation data comprises applying a similarity algorithm and/or a cost function. For example, the similarity algorithm may comprise summing up the amounts of the differences between all corresponding parts, e.g. all pixels, of the compared data. The similarity algorithm may comprise the cost function. For example, the similarity algorithm may comprise an algorithm that is configured to look for the smallest quadratic error in the deviation between the reference data and he measured data. For example, the cost function may comprise minimizing an amount of the comparison. The similarity algorithm may be a trained machine learning algorithm, in particular a trained neural network.

According to an embodiment, determining the scatter correction comprises applying a trained machine learning algorithm at least on the outside scattered radiation data and, optionally, also on the inside radiation data, wherein the output of the machine learning algorithm is in particular estimated inside scattered radiation data and/or scatter-corrected inside radiation data. The trained machine learning network may be deep-learning based. The trained machine learning network may be a neural network, in particular a convolutional neural network. The machine learning algorithm is in particular trained to output the inside scattered radiation data and/or scatter-corrected inside radiation data based on the input of the outside scattered radiation data and/or the inside radiation data. Advantageously, it has been found that application of a machine learning algorithm may provide particularly good results. Since the application is based on the measured outside scattered radiation data, the computational power required can be lower than what would be required for a complete simulation of a scatter pattern. This embodiment may, in general, be used with different kinds of x-ray-based imaging modalities. In particular, this embodiment may be used with computed tomography systems of the 3^{rd} generation, i.e. having a rotating gantry, and with static computed tomography systems. This embodiment can be particularly advantageous for static computed tomography, in particular when comprising a ring detector, since there will usually be scattered radiation reaching the detector elements outside of the primary x-ray beam anyway, that just needs to be recorded in order to be able to apply this embodiment. Similarly, when using collimation in fluoroscopy, there is typically an area of the detector that is not illuminated directly by the primary x-ray beam. Information from the detector, in particular detector elements, outside of the partial area may help to improve the scatter estimation done with the trained machine learning algorithm, e.g. in case it is applied to unknown data.

According to an embodiment, the trained machine learning algorithm has been trained with training data comprising measured input training data and ground truth training data.

Preferably, the input training data may have been measured with a wider collimation of the x-ray beam than the ground truth training data. Additionally or alternatively, the trained machine learning algorithm has been trained with training data comprising simulated data, wherein scattered radiation is simulated. Both, measured training data and simulated training data, may be combined. Combining both may provide a larger amount of training data and, thus, a more reliable and/or accurate trained machine learning algorithm. The measured training data may have been measured with two different collimations of the x-ray beam, such that the input training data has been measured with a wider collimation of the x-ray beam than the ground truth training data. For measuring the training data, for both collimations, the intensity data inside and outside the primary x-ray beam may be collected. Advantageously, the data acquired with the narrow collimation contains lower amount of scattered radiation than the data acquired with the wider collimation. After training, the trained machine learning algorithm may advantageously be able to map from input data, the input data comprising the inside radiation data, to output data, the output data comprising the inside radiation data without the inside scattered radiation data (i.e. the inside radiation data free of scatter). The training data may, for example, correspond to the library data described herein. Additionally or alternatively, the machine learning algorithm may be trained with simulated data. For example, the outside scattered radiation data of the training data may be simulated by Monte Carlo simulations.

According to an embodiment, the trained algorithm is applied for a sub-group of rows of the detector elements of the detector, in particular for a single row of the detector elements. Rows of the detector elements correspond to consecutive detector elements viewed in the circumferential direction of the detector. The rows may correspond to rows of pixels of the recorded detector data. The rows may also be denoted as ring rows. Advantageously, by using a narrow collimation for generating the ground truth training data describing the scatter-free inside radiation data, the ground truth training data can be more scatter-free the narrower the collimation is. Hence, the training data may be better suitable if a narrower collimation is used. Thus, the training data may only correspond to some rows of detector elements the detector, or in the most extreme case, to only one row. Accordingly, the machine learning algorithm may be trained to be applied only to corresponding sub-groups. The machine learning algorithm may then be applied multiple times, i.e. at least once for each sub-group of rows, optionally for each single row. This may lead to overall better results when applying the trained machine learning algorithm.

According to an embodiment, by estimating the part of the inside radiation data that is due to scattered radiation, inside scattered radiation data is determined, wherein the scatter correction is applied by subtracting the inside scattered radiation data from the inside radiation data. Hence the part of the inside radiation data that is estimated to be due to scattered radiation may be subtracted. An alternative may be to directly estimate the scatter-corrected inside radiation data. Determining the inside scattered radiation data and subtracting it may have the advantage that the general structure of the "real" (i.e. non-scattered) data is not directly emulated, which may lead to a loss of information. The original signal may have a higher resolution (e.g. providing a fine bone structure) than the scatter estimate. Data generation by a trained machine learning algorithm may have the disadvantage of comprising convolutions, which may lead to a loss of resolution (e.g. by smoothing the data). On the other hand, scatter signals often tend to be relatively smooth and/or low-frequent signals themselves. Hence, the algorithm does not need to restore the full resolution properties of the input data. Accordingly, this embodiment may ensure that the resolution of the original data is not destroyed or diminished by the application of the algorithm.

The embodiments described herein may be combined with each other unless indicated otherwise. For example, the application of a trained machine learning algorithm and the subtracting of the inside scattered radiation data from the inside radiation data may be combined to achieve a particular advantage.

According to a further aspect of the invention, a method for generating an x-ray-based image, in particular a computed tomography image or a fluoroscope image, is provided. The method comprises applying the method for determining a scatter correction for an x-ray radiation signal recorded by an x-ray detector of an x-ray-based imaging modality, such as a computed tomography system or a fluoroscope. Furthermore, the method comprises applying the scatter correction to generate scatter corrected inside radiation data, and reconstructing an x-ray-based image, in particular computed tomography image or fluoroscope image, in particular reconstructing the x-ray-based image from the scatter-corrected inside radiation data. All features and advantages of the method for determining a scatter correction may be adapted to the method for generating an x-ray-based image and vice versa.

According to a further aspect of the invention, a computer program comprising instructions which, when the program is executed by a computer, such as a control station of an x-ray-based imaging modality, causes the computer and/or the imaging modality to carry out any one of the methods as described herein. All features and advantages of the method for determining a scatter correction and of the method for generating an x-ray-based image may be adapted to the computer program and vice versa.

According to a further aspect of the invention, a computer-readable storage medium, in particular non-transient storage medium, is provided. The computer-readable storage medium comprises instructions which, when executed by a computer, such as a control station of an x-ray-based imaging modality, cause the computer and/or the imaging modality to carry out any one of the methods as described herein. All features and advantages of the method for determining a scatter correction, of the method for generating an x-ray-based image, and of the computer program may be adapted to the computer-readable storage medium and vice versa. The storage medium may be any computer-readable storage medium. For example, the storage medium may be an optical storage medium or a solid-state storage medium. The storage medium may, be a hard-drive, a solid-state-disk a flash storage an online server etc.

According to a further aspect of the invention, an image processing device is provided. The image processing device is configured to perform the steps of the method for determining a scatter correction as described herein. For example, the image processing device may be a computer, a smartphone table, a server, a control station of an imaging modality, such as a computed tomography system or fluoroscope etc. All features and advantages of the method for determining a scatter correction, of the method for generating a computed tomography image, of the computer program, and of the computer-readable storage medium may be adapted to the image processing device and vice versa.

According to a further aspect of the invention, an x-ray-based imaging modality, such as a computed tomography system or a fluoroscope, is provided. The imaging modality comprises a processing circuitry that is configured to carry out the method as described herein. The processing circuitry may be part of a control station of the imaging modality. The computed tomography system may comprise any other components of a computed tomography system, in particular a gantry, an x-ray generating device (comprising at least one x-ray source) configured to generate a primary x-ray beam, and an x-ray detector. In particular, the computed tomography system may comprise any components as described herein with respect to a computed tomography system. The fluoroscope may comprise any other components of a fluoroscope, in particular an x-ray source configured to generate a primary x-ray beam, an x-ray detector, and a collimator. In particular, the fluoroscope may comprise any components as described herein with respect to a fluoroscope and/or components that are typically part of a fluoroscope. All features and advantages of the method for determining a scatter correction, of the method for generating an x-ray-based image, of the computer program, of the computer-readable storage medium, and of the image processing device may be adapted to the x-ray-based imaging modality and vice versa.

According to a further aspect of the invention, a use of a imaging data acquired by an x-ray detector of an x-ray-based imaging modality outside of a primary x-ray beam of the imaging modality to determine a scatter correction for imaging data acquired by the x-ray detector within the primary x-ray beam is provided. In particular, the imaging data acquired by a detector of a x-ray-based imaging modality outside of a primary x-ray beam may have been acquired at the same time as the imaging data the scatter correction is determined for. Advantageously, in particular without requiring any further measurements, a relatively simple and efficient way of determining a scatter correction may be achieved by this use. All features and advantages of the method for determining a scatter correction, of the method for generating an x-ray-based image, of the computer program, of the computer-readable storage medium, of the image processing device, and of the x-ray-based imaging modality may be adapted to the use of computed tomography data and vice versa.

The accompanying drawings illustrate various exemplary embodiments and methods of various aspects of the invention.
Fig. 1 shows an x-ray detector and an arrangement of x-ray sources according to an embodiment of the invention in a view facing the axial direction;
Fig. 2 shows an x-ray detector and an arrangement of x-ray sources according to the embodiment of figure 1 in a side view;
Fig. 3 shows an x-ray detector and an arrangement of x-ray sources according to the embodiment of the invention of figure 1 and 2 in a view facing the axial direction;
Fig. 4 shows a flow diagram of a method for determining a scatter correction for an x-ray radiation signal recorded by an x-ray detector of a computed tomography system according to an embodiment of the invention;
Fig. 5 shows a flow diagram of a method for generating a computed tomography image according to an embodiment of the invention;
Fig. 6 shows a schematic overview of a fluoroscope with an x-ray detector and an x-ray source according to an embodiment of the invention;
Fig. 7 shows a flow diagram of a method for determining a scatter correction for an x-ray radiation signal recorded by an x-ray detector of a fluoroscope according to an embodiment of the invention;
Fig. 8 shows a schematic view of radiation data comprising inside radiation data within a partial area and outside scattered radiation data outside of the partial area;
Fig. 9 shows a plot of a radiation data line profile from figure 8;
Fig. 10 shows a plot of another radiation data line profile from figure 8;
Fig. 11 shows outside scattered radiation data of the radiation data line profile of figure 9;
Fig. 12 shows inside scattered radiation data of the radiation data line profile of figure 9;
Fig. 13 shows an estimation of the scattered radiation within the partial area in the form of a line profile; and
Fig. 14 shows the estimation of figure 13 together with the scatter line plots of figure 11.

Similar elements are designated with the same reference signs in the drawings.

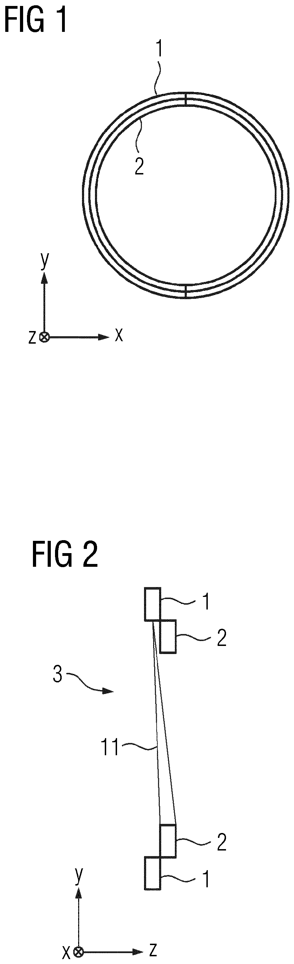

Figure 1 shows an x-ray detector 2 and an arrangement of x-ray sources 1 of a computed tomography system according to an embodiment of the invention in a view facing the axial direction. The axial direction corresponds to the z-direction. The radial directions correspond to the x,y-directions. The arrangement of x-ray sources 1 is arranged around an examination area 3. The x-ray detector 2 is an x-ray detector ring 2 comprising multiple detector elements that are arranged around the examination area 3. Viewed in radial direction, the x-ray detector ring 2 is positioned closer to the examination area 3 than the x-ray source ring 1.

Figure 2 shows an x-ray detector 2 and an arrangement of x-ray sources 1 of a computed tomography system according to the embodiment of figure 1 in a side view, i.e. facing in a radial direction, namely, here, in x-direction. As can be seen in this view, the x-ray source ring 1 and the x-ray detector ring 2 are slightly shifted with respect to each other in the axial direction, i.e. in z-direction. In addition to the x-ray detector ring 2 and the x-ray source ring 1, the primary x-ray beam 11 is also indicated in this side view. The primary x-ray beam 11 is emitted by an x-ray source and is transmitted directly to the opposing detector elements of the detector ring 2, thereby illuminating these detector elements. The shift in z-direction between the x-ray detector ring 2 and the x-ray source ring 1 allows for the x-ray beam 11 to reach the opposing detector elements without being blocked by the detector elements on the same side as the emitting x-ray sources.

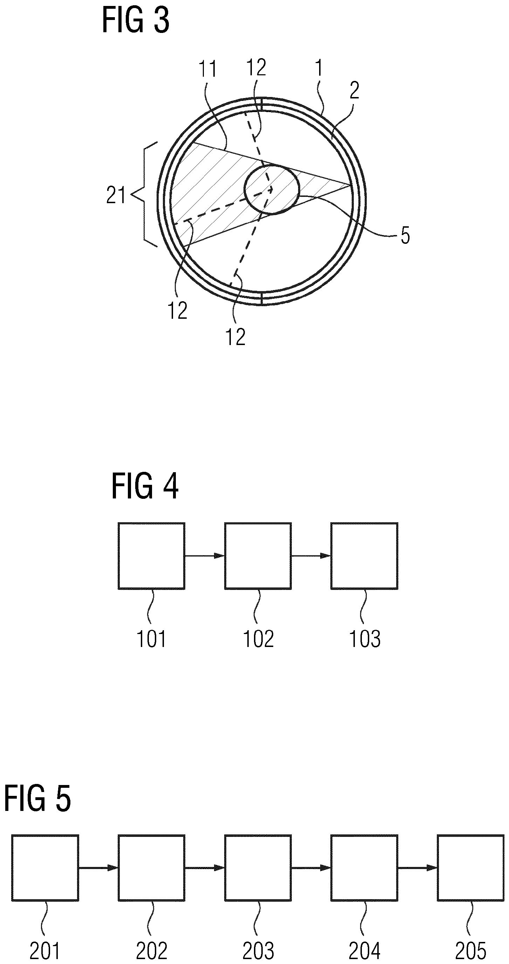

Figure 3 shows an x-ray detector 2 and an arrangement of x-ray sources 1 of a computed tomography system according to the embodiment of the invention of figure 1 and 2 in a view facing the axial direction. In this representation, the primary x-ray beam 11 as well as an object 5 that is examined are indicated. The primary x-ray beam 11 is a cone-shaped beam and the partial area 21 of the detector elements that are directly illuminated by the primary x-ray beam 11 is determined by the cone shape of the primary x-ray beam. Additionally, the directions of a few exemplary cases of scattered radiation 12 are indicated by dashed lines. Hence, there is scattered radiation reaching the detector elements within the partial area 21 and outside the partial area 21. The scattered radiation 12 reaching the detector elements within the partial area 21 may disturb the CT measurement of the object, e.g. leading to image artifacts. On the other hand, according to this invention, the scattered radiation 12 reaching the detector elements outside the partial area 21 may be used to determine the scattered radiation within the partial area 21 to apply a scatter correction. Advantageously, it can be assumed that radiation measured outside the partial area 21 is purely due to scattered radiation.

Figure 4 shows a flow diagram of a method for determining a scatter correction for an x-ray radiation signal recorded by an x-ray detector 2 of a computed tomography (CT) system according to an embodiment of the invention. The computed tomography system may, for example, comprise an x-ray detector ring 2 and an x-ray source ring 1 as shown in figures 1-3. In a first step 101, a partial area 21 of the x-ray detector that comprises a subset of the detector elements of the detector 2 is illuminated with a primary x-ray beam 11. The primary x-ray beam 11 is directed at an object within the examination area 3. The detected x-ray radiation within the partial area 21 is recorded as inside radiation data. Furthermore, in another step 102, scattered radiation is detected with at least some of the detector elements outside of the directly illuminated partial area 21 as outside scattered radiation data. These two steps 101, 102 may preferably be performed simultaneously. For example, having a static CT system, the x-ray sources of the x-ray source ring 1 may be activated one after the other to consecutively generate primary x-ray beams 11 from different angles. For each of these x-ray beam 11, the inside radiation data and the outside scattered radiation data may be recorded and the following scatter correction may be applied for each primary x-ray beam 11. Optionally, these first steps 101, 102 may be replaced by a step of receiving inside radiation data and outside scattered radiation data. Hence, the creation of the data does not necessarily have to be part of the method. For example, the inside radiation data and outside scattered radiation data may be received from a database. For example, the inside radiation data and outside scattered radiation data may be received from a computed tomography imaging system. In this case, the following step 103 may thus be performed with the received data. In the following step 103, a scatter correction is determined based on the outside scattered radiation data that was recorded in step 102. For example, determining the scatter correction may comprises applying a trained machine learning algorithm, such as a trained neural network, at least on the outside scattered radiation data and, optionally, also on the inside radiation data. The output of the machine learning algorithm may be scatter-corrected inside radiation data. Alternatively, the output of the machine learning algorithm may be an estimate of the inside scattered radiation data, i.e. the scattered radiation that has reached the detector elements within the partial area 21. The trained machine learning algorithm may be trained with training data comprising measured input training data and ground truth training data. The input training data may be measured with a wider collimation of the x-ray beam than the ground truth training data. In this case the trained algorithm may preferably be applied for a sub-group of rows of the detector elements of the detector 2, in particular for a single row of the detector elements. Additionally or alternatively, the training data may comprise simulated data wherein scattered radiation is simulated. Hence, the scatter correction may be determined by estimating the part of the inside radiation data that is due to scattered radiation based on the outside scattered radiation data. The inside scattered radiation data may be subtracted from the inside radiation data in order to perform the scatter correction. Next to the application of the machine learning algorithm, there are other variants that may also be applied to determine the inside scattered radiation data. In particular a calculation model and/or an algorithm may be applied that derives the scattered radiation within the partial area 21 from the outside scattered radiation data. According to one alternative, the scattered radiation may be determined by interpolating from the outside scattered radiation data. Optionally, for this purpose, a structure of scattered radiation outside the partial area 21 may be determined from the outside scattered radiation data, and the structure of scattered radiation may be interpolated and/or inpainted into the partial area 21. According to another option, the scatter correction may be determined by comparing the outside scattered radiation data to a database comprising a library of stored examples of outside scattered radiation data, the stored examples being associated with inside scattered radiation data, respectively. In this example, the scatter correction may be determined based on the inside scattered radiation data.

Figure 5 shows a flow diagram of a method for generating a computed tomography image according to an embodiment of the invention. The first steps 201-203 may correspond to the steps 101-103 as described with respect to the embodiment of the method as shown in figure 4. Furthermore, the method comprises another step 204 of applying the scatter correction to generate scatter corrected inside radiation data, and another step 205 of reconstructing a computed tomography image, in particular reconstructing the computed tomography image from the scatter-corrected inside radiation data.

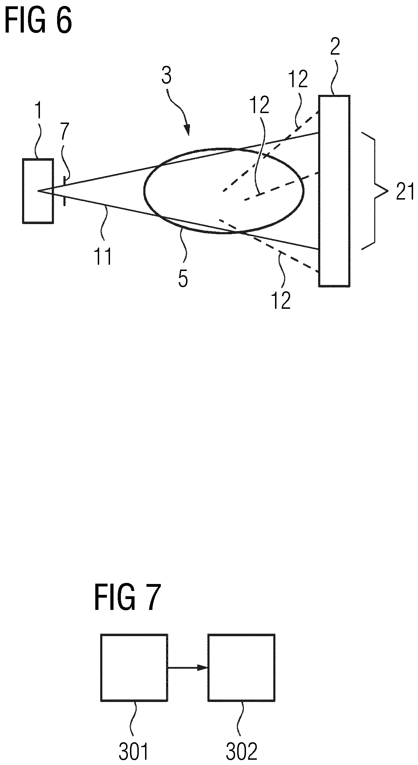

Figure 6 shows a schematic overview of a fluoroscope with an x-ray detector 2 and an x-ray source 1 according to an embodiment of the invention. For example, the fluoroscope may be a c-arm fluoroscope, e.g. the x-ray detector 2 and the x-ray source may be connected by the c-arm. In between the x-ray source 1 and the x-ray detector is an examination area 3, where an object 5 is placed that is illuminated by a primary x-ray beam 11. The primary beam 11 also illuminates a partial area 21 of the x-ray detector 2. The size and position of the partial area 21 is determined by a collimator 7 that collimates the primary x-ray beam 11. Due to scattering inside the object 5, scattered radiation 12 is hitting the x-ray detector 2 inside and outside the partial area 21.

Figure 7 shows a flow diagram of a method for determining a scatter correction for an x-ray radiation signal recorded by an x-ray detector 2 of a fluoroscope according to an embodiment of the invention. The fluoroscope may, for example, comprise an x-ray detector 2 and an x-ray source 1 as shown in figure 6.

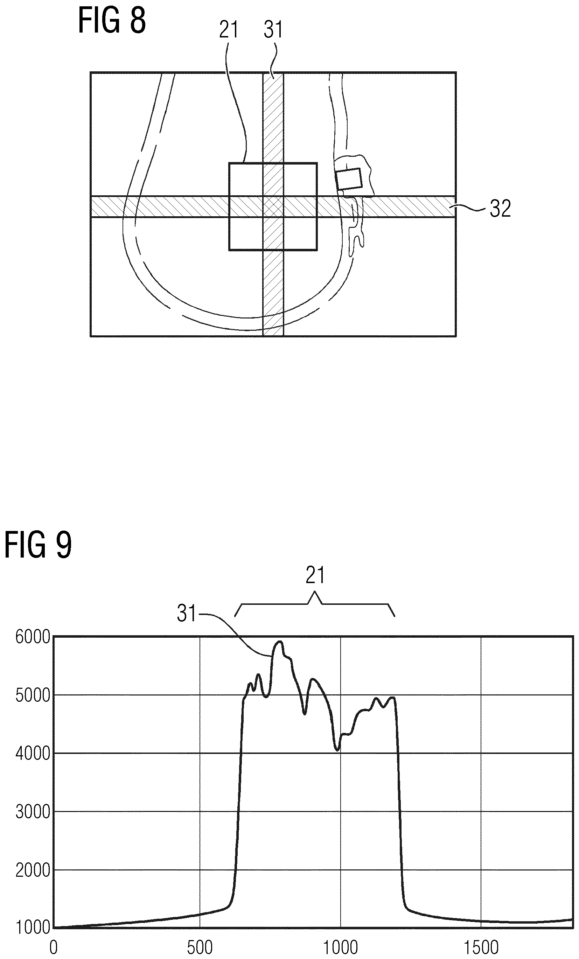

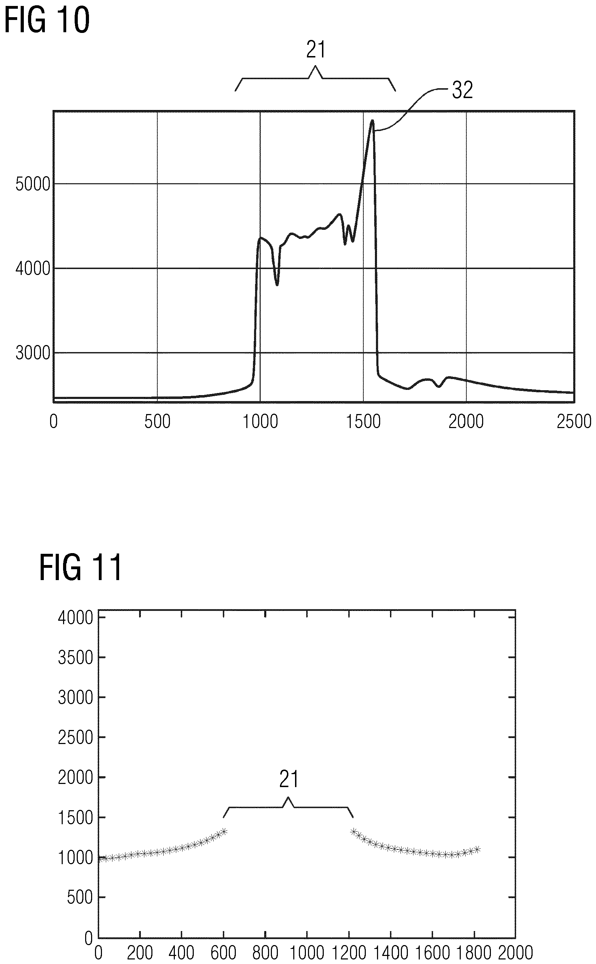

In a first step 301, inside radiation data and outside scattered radiation data is received. The inside radiation data is data obtained from a directly illuminated partial area 21 of the x-ray detector 2 with a primary x-ray beam 11 that is directed at an object 5 within the examination area 3. The shape of the primary x-ray beam 11 and the partial area is defined by a collimator 7. The outside scattered radiation data is data obtained from detecting scattered radiation 12 outside of the directly illuminated partial area 21. In a further step 302, based on the outside scattered radiation data, a scatter correction is determined. For this purpose, a part of the inside radiation data that is due to scattered radiation 12 is estimated. The estimation of the scattered radiation inside the partial area 21 may be performed as indicated in figures 8 to 14. Figure 8 shows a schematic view of radiation data comprising inside radiation data within a partial area 21 and outside scattered radiation data outside of the partial area 21. Furthermore, a first radiation data line profile 31 going vertically and a second radiation data line profile 32 going horizontally is indicated. Hence, the first radiation data line profile 31 and the second radiation data line profile 32 are perpendicular to each other. The line profiles 31, 32 are averaged over their respective indicated width. A plot of the first radiation data line profile 31 is shown in figure 9 and a plot of the second radiation data line profile 32 is shown in figure 10.

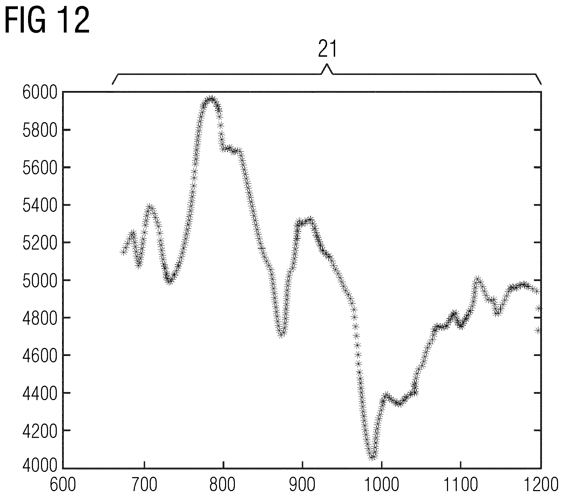

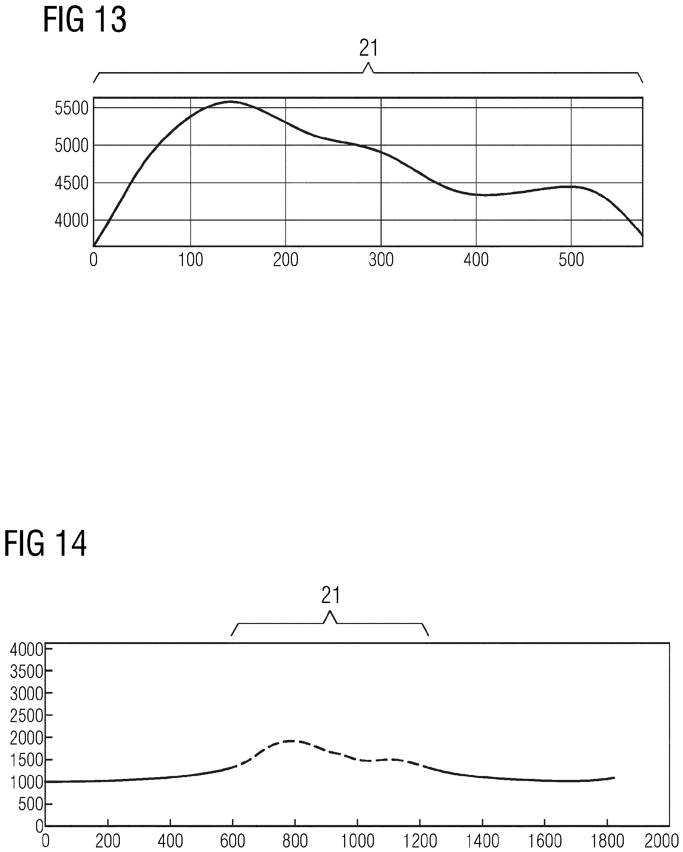

Figure 11 shows the parts of the first radiation data line profile 31 that is outside the partial area 21, corresponding to outside scattered radiation data, and figure 12 shows the parts of the first radiation data line profile 31 that are within the partial area 21, corresponding to inside radiation data. The inside radiation data, as shown in figure 12 comprises both, scattered radiation 12 and radiation from the primary x-ray beam 11. The outside scattered radiation data as shown in figure 11 is based only on scattered radiation 12. The two parts of the first radiation data line profile 31 as shown in figure 11 correspond to scatter line plots at two opposite sides of the partial area 21. Correspondingly, there may also be two parts of the second radiation data line profile 32 that correspond to scatter line plots at two other opposite sides of the partial area 21. Figure 13 shows an estimation of the scattered radiation within the partial area 21 in the form of a line profile. The estimation is based on the line profiles of figures 11 and 12. Figure 14 shows the estimation of figure 13 together with the scatter line plots of figure 11. Hence, Figure 14 corresponds to the scattered radiation within and outside of the partial area. As can be seen, the scatter estimation within the partial area 21 (dashed line) is continuous and differentiable to the profile lines outside the partial area 21 (solid lines). Furthermore, the scatter estimation within the partial area 21 is an adaption of the signal as shown in figure 12 but with a blurred, i.e. smoothed course. Hence, for example, compared to figure 9, there is no jump in the signal line and the signal within the partial area is smoother. Advantageously, due to the boundary conditions, including continuous and differentiable line plot with respect to the scatter line plots, the scatter intensity inside the partial area 21 can be partially based on real conditions and partially on estimations. The scatter estimation may further be determined based on other scatter line plots such as the scatter line plots of second radiation data line profile 32 that is shown in figure 9. Hence, for example, using the vertical line plots from figure 9 and the horizontal line plots from figure 10, line plots from four different sides of the partial area 21 may be used to estimate the part of the inside radiation data that is due to scattered radiation 12.

## Claims

1. A method for determining a scatter correction for an x-ray radiation signal recorded by an x-ray detector (2) of an imaging modality based on x-ray radiation,
the method comprising the following method steps:
(a) receiving inside radiation data and outside scattered radiation data,
wherein the inside radiation data is data obtained from a partial area (21) of the x-ray detector (2), wherein the partial area (21) is directly illuminated with a primary x-ray beam (11), wherein the primary x-ray beam is directed at an object within an examination area (3), wherein the outside scattered radiation data is data obtained by detecting scattered radiation (12) outside of the partial area (21),
(b) determining the scatter correction based on the outside scattered radiation data, in particular by estimating a part of the inside radiation data that is due to the scattered radiation (12).

2. The method according to claim 1,
wherein the method further comprises as part of receiving the inside radiation data and the outside scattered radiation data:
- directly illuminating the partial area (21) of the x-ray detector (2) with the primary x-ray beam (11) directed at the object within the examination area (3),
- recording the inside radiation data by detecting the x-ray radiation within the partial area (21),
- recording the outside scattered radiation data by detecting the scattered radiation (12) with a part of the detector outside of the directly illuminated partial area (21), in particular with at least some of the detector elements of the detector outside of the directly illuminated partial area (21).

3. The method according to any one of the preceding claims, wherein a structure, such as at least one scatter line profile or a two-dimensional scatter distribution, of the scattered radiation (12) outside the partial area (21) is determined from the outside scattered radiation data, and the structure of the scattered radiation (12) is interpolated and/or inpainted into the partial area (21) in order to estimate the part of the inside radiation data that is due to the scattered radiation (12).

4. The method according to claim 3,
wherein the structure is interpolated from at least four different sides of the partial area.

5. The method according to claim 3 or 4,
wherein at least one pair of signal line plots is interpolated from scatter line plots outside the partial area into the partial area under the condition that the interpolation is continuous and, preferably, differentiable.

6. The method according to any one of the preceding claims, wherein determining the scatter correction is further based on one or several of the following:
- previously measured and/or simulated scatter data from within and/or outside the partial area (21);
- a geometric measure, in particular thickness, of the object;
- the inside radiation data, in particular a distribution and/or signal strength of the inside radiation data.

7. The method according to any one of the preceding claims, wherein the scatter correction is determined by comparing the outside scattered radiation data to a database of stored examples of outside scattered radiation data, the stored examples being associated with inside scattered radiation data, respectively, and the scatter correction is determined based on the inside scattered radiation data.

8. The method according to any one of the preceding claims,
wherein determining the scatter correction comprises applying a trained machine learning algorithm at least on the outside scattered radiation data and, optionally, also on the inside radiation data,
wherein the output of the machine learning algorithm is in particular estimated inside scattered radiation data and/or scatter-corrected inside radiation data.

9. The method according to claim 8,
wherein the trained machine learning algorithm has been trained with training data comprising measured input training data and ground truth training data, the input training data being measured with a wider collimation of the x-ray beam than the ground truth training data; and/or
the training data comprising simulated data, wherein scattered radiation (12) is simulated.

10. The method according to claim 8 or 9,
wherein the trained algorithm is applied for a sub-group of rows of the detector elements of the detector (2), in particular for a single row of the detector elements.

11. The method according to any one of the preceding claims,
wherein, by estimating the part of the inside radiation data that is due to scattered radiation (12), inside scattered radiation data is determined,
wherein the scatter correction is applied by subtracting the inside scattered radiation data from the inside radiation data.

12. A computer program comprising instructions which, when the program is executed by a computer, such as a control station of an imaging modality, causes the computer to carry out the method according to any one of the preceding claims.

13. An image processing device that is configured to perform the method according to any one of claims 1 to 11.

14. An x-ray-based imaging modality, in particular a computed tomography system or a fluoroscope, comprising a processing circuitry that is configured to carry out the method according to any one of claims 1 to 11.

15. A use of imaging data acquired by an x-ray detector (2) of an x-ray-based imaging modality outside of a primary x-ray beam (11) of the imaging modality to determine a scatter correction for imaging data acquired by the x-ray detector (2) within the primary x-ray beam (11).
